Europäisches Patentamt

European Patent Office

Office européen des brevets

(11) Publication number: **0 360 565**
A1

## EUROPEAN PATENT APPLICATION

(21) Application number: **89309528.1**

(22) Date of filing: **19.09.89**

(51) Int. Cl.⁵: **B 01 D 61/00**
B 01 J 20/00
// A61L15/00, F26B5/16

(30) Priority: **22.09.88 US 247828**

(43) Date of publication of application:
**28.03.90 Bulletin 90/13**

(84) Designated Contracting States: **ES GR**

(71) Applicant: **ALLIED-SIGNAL INC.**
**Columbia Road and Park Avenue P.O. Box 2245R**
**Morristown New Jersey 07960 (US)**

(72) Inventor: **White, Lloyd S.**
**2130 S. Goebbert Road No. 206**
**Arlington Heights Illinois 60005 (US)**

(74) Representative: **Brock, Peter William et al**
**URQUHART-DYKES & LORD 91 Wimpole Street**
**London W1M 8AH (GB)**

(54) Osmotic absorbents.

(57) Osmotic absorbents which possess the ability to absorb liquids to a high degree comprise encapsulated semipermeable polymeric membrane shells containing therein a high osmolality promoter, the ratio of polymer to promoter being in a range of from about 2:1 to about 1:10. The absorbents may be characterized by a shell of cellulose acetate having sodium chloride contained therein.

EP 0 360 565 A1

**Description**

## OSMOTIC ABSORBENTS

### BACKGROUND OF THE INVENTION

Most natural and synthetic fibers possess a limited capacity for the storage of liquid; however, if a fiber is to store liquids in a useful manner, the fiber cannot release this liquid upon being subjected to any stress. Such materials will find a wide variety of uses where absorption of liquids is required. For example, fibers with absorbent properties are used in disposable absorbent products. These products will include diapers, feminine hygiene products, hospital disposables, surgical dressings, and industrial wipes, the largest market for these disposable absorbent products being baby diapers. Currently, disposable diapers use either wood fluff as an absorbent or a combination of superabsorbent polymer gel and wood fluff.

Wood fluff is comprised of a blend of wood fibers in which the individual fibers possess cross-section dimensions ranging from about 20 to about 100 microns, usually in the form of long, thin ribbons. Densities of the individual fibers are in a range of from about 0.9 to about 1.1 g/cm$^3$. However, wood fluff when used as an absorbent mat in diapers has a preferred bulk density of 0.08 g/cm$^3$. This difference in density creates a large void volume between the individual fibers where absorbed liquid is stored. However, since this liquid is held by the capillary forces on the external surfaces of the fibers, it can easily be dislodged by stress. It has been reported that the capacity of wood fluff may drop from about 10 g/g to about 1 g/g under a 1,000 G stress. Therefore, it is readily apparent that diapers utilizing wood fluff as an absorbent may lose the liquid content and thus create various and sundry problems.

The osmotic absorbent described in these claims is an expanding membrane system that encapsulates water within a membrane shell. This volume expansion is an important aspect of this invention in that it is the mechanism by which greater absorption capacity is obtained. Compared to static nonexpanding structures such as wood fluff, volume expansion due to osmotic pressures creates space for uptakes of up to 2100 wt.% or greater observed with these osmotic absorbents.

Superabsorbent polymer gels (AGM's) used in diaper applications are high molecular weight ionic polymers with a small percentage of cross-links. Control of this cross-link density in AGM's causes both water-insoluble particles and swelling capacity for volume expansion. U.S. Patent 4,703,067 describes a process for preparing a solid, water absorbing, cross-linked polyacrylate resin via polymerization without external heating. U.S. Patent 4,677,174 describes water absorbing cross-linked acrylate resin copolymers prepared by aqueous polymerization of various monomers. In neither case are membrane structures incorporated as with these osmotic absorbents. The osmotic absorbents of this invention include the advantageous aspects of AGM's in being volume expanding systems.

AGM's are typically dispersed as granules supported by the wood fluff matrix within a diaper core. The thin ribbon-like nature of the wood fibers help in the acquisition and distribution of liquid within a diaper core, while AGM acts as the vehicle for irreversible storage. Therefore, another advantage to osmotic absorbents of this invention is the improved irreversible liquid storage.

These osmotic absorbents contain a membrane component. This makes liquid absorption into the shell a diffusion-controlled process, where molecules diffuse across an osmotic gradient pathway. Since external pressures and forces are not required and are not introduced, flow rates will be substantially lower than those realized in conventional reverse osmosis (RO) membrane operation. In some respects, i.e. low or non-existent applied pressure drops, the osmotic absorbents of this invention involve a dialysis process. Dialysis has been defined as any process in which the principle effect is the transfer of solute molecules from one liquid to another through a membrane when the important differences in the state of the liquids are the chemical potentials of their solutes. Conventional dialysis membranes, such a those used in hemodialysis, have molecular weight cutoffs of 1000 atomic mass units or more. Osmotic pressure is a number-dependent phenomena; therefore, high flow rates from an osmotic pressure difference require large numbers of molecules per unit volume. Therefore, the promoter component of these osmotic absorbents is a low molecular weight species under 500 atomic mass units for both weight and volume efficiency. In contradistinction from dialysis, these osmotic absorbents use a membrane component with RO or hyperfiltration qualities.

As will hereinafter be shown in greater detail, it has now been discovered that an osmotic absorbent may be prepared which will absorb many times its weight in liquid, the amount of liquid being absorbed being greater than that which is absorbed in prior absorbents hereinbefore discussed.

### BRIEF SUMMARY OF THE INVENTION

As was previously discussed, disposable absorbent materials or products will find a wide variety of uses in the commercial field. Heretofore, these absorbent products usually consisted of compositions such as wood fluff or a combination of a polymer gel and wood fluff. The liquid retentive properties of these products constitute an important characteristic of the object, the term "liquid retentive" including both the amount of liquid as well as the reversibility of the retention. As will hereinafter be shown in greater detail, it has now been discovered that osmotic absorbents may be prepared which will possess the ability to absorb and retain liquid in amounts up to over 2100% of the weight of the total absorbent.

It is therefore an object of this invention to provide an absorbent.

A further object of this invention is to provide osmotic absorbents which possess the ability to irreversibly absorb liquids in an amount greater in excess of the weight of the absorbent.

In one aspect, an embodiment of this invention resides in an osmotic absorbent comprising an encapsulated semipermeable polymeric membrane shell containing therein a high osmolality promoter.

A specific embodiment of this invention is found in an osmotic absorbent comprising an encapsulated semipermeable polymeric membrane shell of cellulose acetate, the thickness of said membrane shell being in a range of from about 5 to about 30 microns containing therein a high osmolality promoter comprising sodium chloride.

## DETAILED DESCRIPTION OF THE INVENTION

As hereinbefore set forth, the present invention is concerned with osmotic absorbents. These products comprise an encapsulated semipermeable polymeric membrane shell containing therein a high osmolality promoter. These absorbents will find a wide variety of uses. For example, the materials which possess high uptake values may be used in disposable absorbent products such as baby diapers. Diapers are usually designed to absorb urine which is a weak salt solution having a strength of about 1%. Currently, diaper designs utilize wood fluff admixed with superabsorbent polymers. The most popular polymers currently in use comprise derivatives of cross-linked polyacrylic acid and are commonly known as absorbent gelling material (AGM). When measured against urine solutions, and in view of other given design constraints, liquid uptake by the AGM is limited to about 30 times by weight. The mechanism of AGM swelling is primarily by osmotic pressure; however, it is limited by the weight percent of the osmotic promoter which can be incorporated within the structure. In contradistinction to this, the osmotic absorbents of the present invention employ an osmotic or high osmolality promoter in a concentration that can be increased at will. As will hereinafter be shown in greater detail, various types of high osmolality promoters may be employed, which is in contrast to the limitation of utilizing ionic species as the osmotic promoter with AGM. Therefore, in view of the fact that the high osmolality of the promoters of the present invention are not so limited, the osmotic absorbent of the present invention will be found to have absorption capacities which greatly exceed that of the AGM.

While the above discussion has been centered upon the use of osmotic absorbents in diaper application, it is also contemplated within the scope of this invention that said absorbents may also be utilized in other ways, according to the particular polymeric membrane shell and osmolality promoter which is employed. For instance, the absorbents of the present invention may also be utilized to remove unwanted liquids from hydrocarbon fuels wherein the presence of these liquids, such as water, will have a deleterious affect upon the use of said fuels.

The osmotic absorbence of the present invention will, as previously set forth, comprise an encapsulated semipermeable polymeric membrane shell containing enclosed therein a high osmolality promoter. The semipermeable membranes which may be employed as the shell component of the absorbent will comprise those polymeric membranes which possess good water permeability combined with a high rejection of the osmotic promoter. Membranes which possess these properties will permit an excellent water or liquid flux into the membrane shell while building a high osmotic pressure difference. Some representative examples of polymeric membranes will comprise those prepared from cellulose acetate including di- and triacetates, cellulose acetate derivatives such as methylcellulose, ethylcellulose, prapylcellulose, nitrocellulose, etc., sulfonated polysulfone, polyelectrolyte complexes such as a complex of polystyrene sulfonate and polybenzyl trimethyl ammonium chloride, long chain polymeric amides, including the various nylons such as nylon 4, nylon 6, nylon 7, nylon 8, nylon 9, nylon 66, etc., interpenetrating polymer network membranes such as those prepared by reacting a polyether such as polyethylene glycol with an isocyanate such as toluene diisocyanate to form an isocyanate-capped polyether, and thereafter admixing said polyether with a heterocyclic nitrogen-containing compound such as polyvinyl pyrridine, membranes formed by reacting an aromatic polyamine such as m-phenylene diamine with an aromatic polycarboxylic acid chloride such as trimesoyl chloride to form an interfacial polymerized polymer. While the polymers may be used as the shell per se, it is also contemplated within the scope of this invention that the polymers may be supported on a porous support backing material such as polysulfone, polycarbonate, etc.

The high osmolality promoter which comprises the second component of the absorbent and which is encapsulated within the polymeric membrane shell will preferably comprise a low molecular weight water-soluble component which also has a good rejection rate by the membrane. Some representative examples of these high osmolality promoters will include halide salts of Groups IA and IIA of the Periodic Table such as lithium chloride, sodium chloride, potassium chloride, rubidium chloride, calcium chloride, barium chloride, magnesium chloride, lithium bromide, sodium bromide, potassium bromide, rubidium bromide, calcium bromide, barium bromide, magnesium bromide, etc., sugars such as glucose, sucrose, fructose, etc., polyhydric compounds such as glycerol, ethylene glycol, ammonium compounds such as ammonium hydroxide, ammonium chloride, etc., lactic acid, potassium bicarbonate, sodium bicarbonate, lithium blcarbonate, etc. It is to be understood that the aforementioned list of polymers for the membrane layer and osmotic promoter are only representative of the class of compounds which may be employed to form the osmotic absorbent and that the present invention is not necessarily limited thereto.

The csmotic absorbents comprising the encapsulated semipermeable polymeric membrane shell containing therein the osmotic promoter will contain a weight ratio of polymer to promoter in a range of from about 2:1 to about 1:10. For a desired rate of uptake of liquid, the amount of osmotic promoter which is utilized will be dependent upon the weight of the polymer. The latter weight will then be dependent upon the thickness of the semipermeable polymeric membrane shell, said thickness, in the preferred embodiment of the invention, being in a range of from about 5 to about 30 microns.

The configuration of the osmotic absorbent may be widely varied in nature. Some representative examples of forms of osmotic absorbents will comprise sheets, spheres, hollow fibers, etc. These absorbents may be prepared by any method known in the art. For example, when the osmotic absorbent is to be in the form of sheets, the osmotic promoter layer may be placed between a top layer and lower layer of the membrane, said layers then being laminated to form an encapsulated shell containing the osmotic promoter encapsulated therein. Likewise, the osmotic promoter, especially when in solid form, may be coated with the desired polymer which is thereafter treated to form the desired membrane. Likewise, if the osmotic absorbent is to be in the form of a hollow fiber, the fiber may be formed of the semipermeable polymeric membrane, again by any manner known in the art, following which the osmotic promoter is loaded inside the length of fibers which are thereafter sealed at each end to prevent leakage.

### EXAMPLE I

In this example, a sheet of polymeric cellulose acetate measuring 3x3 cm and having a thickness of 20 microns was placed on a flat surface. Varying amounts of osmotic promoter comprising glucose and sucrose were placed in the center of the sheet, following which a second sheet of polymeric cellulose acetate was placed on top of the first sheet and osmotic promoter. The edges were sealed by softening of the polymer and each of the packets was weighed. The osmotic absorbent packets comprising the osmolality promoter encapsulated in the polymeric cellulose acetate were then placed in water for period of time ranging from 30 minutes to 360 minutes. Following this, the packets were then removed from the water, pressed dry with an absorbent paper towel, and the soaked weight was obtained. The results of this test are set forth In Table 1 below in which packets A, B, C, and D contain varying amounts of glucose as the osmotic promoter and packet E contained sucrose as the osmotic promoter.

TABLE 1

| Sample Reference | Polymer WGT CA(g) | Promoter ID and WGT(g) | Packet WGT(g) | Time (min) | Final WGT(g) | Uptake on Packet Basis | Uptake on Polymer Basis |
|---|---|---|---|---|---|---|---|
| A | 0.037 | glucose: 0.045 | 0.082 | 30 | 0.230 | 180% | 400% |
| B | 0.036 | glucose; 0.077 | 0.113 | 90 | 0.372 | 229% | 719% |
| C | 0.056 | glucose; 0.054 | 0.110 | 90 | 0.291 | 165% | 323% |
| D | 0.028 | glucose; 0.037 | 0.065 | 90 | 0.257 | 295% | 686% |
| E | 0.056 | sucrose; 0.134 | 0.190 | 360 | 1.031 | 443% | 1502% |

EXAMPLE II

In a manner similar to that set forth in Example I above, osmotic absorbent packets were prepared by encapsulating varying amounts of sodium chloride in semipermeable polymeric membrane shells comprising cellulose acetate, the cellulose acetate having a similar surface area and thickness to those set forth in the above example. The packets which were designated F, G, H, I, and J were immersed in water for a period of time ranging from 90 to 1060 minutes. At the end of the designated time periods, the packets were removed, patted dry with an absorbent paper towel, and weighed. The results of these tests are set forth in Table 2 below.

TABLE 2

| Sample Reference | Polymer WGT CA(g) | Promoter ID and WGT(g) | Packet WGT(g) | Time (min) | Final WGT(g) | Uptake on Packet Basis | Uptake on Polymer Basis |
|---|---|---|---|---|---|---|---|
| F | 0.034 | NaCl; 0.067 | 0.101 | 90 | 0.596 | 490% | 1456% |
| G | 0.050 | NaCl; 0.036 | 0.086 | 350 | 1.042 | 1112% | 1912% |
| H | 0.040 | NaCl; 0.046 | 0.086 | 240 | 0.875 | 917% | 1973% |
| I | 0.251 | NaCl; 0.136 | 0.387 | 300 | 6.221 | 1507% | 2324% |
| J | 0.187 | NaCl; 0.251 | 0.438 | 1060 | 9.616 | 2100% | 4908% |

EXAMPLE III

Four packets were prepared in a manner similar to that set forth in the above examples utilizing sodium chloride, sucrose, and glucose as the high osmolality promoters and cellulose acetate as the semipermeable polymeric membrane. After preparing and sealing the packets three of the four packets were immersed in water and the fourth packet was immersed in a solution containing 1% by weight of sodium chloride. The packets were immersed for periods of time up to 48 hours. During this 48-hour period, the packets were recovered at various intervals, patted dry, and weighed. The results of these tests are set forth in Tables 3 and 4 below. In the tests, packets K, L, and M were those immersed in water while packet N was immersed in the sodium chloride solution.

Table 3

Uptake Based on Promoter Weight

| | K | L | M | N |
|---|---|---|---|---|
| Polymer Weight (g) | 0.187 | 0.056 | 0.212 | 0.158 |
| Promoter Identity | NaCl | Sucrose | Glucose | NaCl |
| Promoter Weight (g) | 0.251 | 0.134 | 0.436 | 0.156 |
| Time in Minutes | | % Uptake | | |
| 0 | 0 | 0 | 0 | 0 |
| 15 | 143 | - | - | - |
| 30 | - | 118 | 115 | 52 |
| 60 | 679 | 216 | 225 | 118 |
| 120 | 1069 | 335 | 405 | 315 |
| 240 | - | - | 608 | 528 |
| 300 | - | - | - | 938 |
| 360 | - | 628 | - | - |
| 1060 | 3657 | - | - | - |
| 1200 | - | - | 1492 | - |
| 1320 | - | 1237 | - | - |
| 1350 | - | - | - | 2188 |
| 2475 | 4354 | - | - | - |
| 2820 | - | - | 2263 | - |
| 2880 | - | 1927 | - | - |

Table 4

Uptake Based on Polymer Weight

| | K | L | M | N |
|---|---|---|---|---|
| Polymer Weight (g) | 0.187 | 0.056 | 0.212 | 0.158 |
| Promoter Identity | NaCl | Sucrose | Glucose | NaCl |
| Promoter Weight (g) | 0.251 | 0.134 | 0.436 | 0.156 |
| Time in Minutes | | % Uptake | | |
| 0 | 0 | 0 | 0 | 0 |
| 15 | 192 | - | - | - |
| 30 | - | 282 | 236 | 51 |
| 60 | 911 | 518 | 462 | 116 |
| 120 | 1434 | 802 | 833 | 311 |
| 240 | - | - | 1251 | 521 |
| 300 | - | - | - | 927 |
| 360 | - | 1502 | - | - |
| 1060 | 4908 | - | - | - |
| 1200 | - | - | 3069 | - |
| 1320 | - | 2959 | - | - |
| 1350 | - | - | - | 2162 |
| 2475 | 5844 | - | - | - |
| 2820 | - | - | 4655 | - |
| 2880 | - | 4611 | - | - |

It is to be noted from the above tables that each packet had a percentage uptake based on the promoter weight of about 2,000% or more while the uptake of water based on polymer weight ranged from 2,162% of the sodium chloride solution to over 4,600 wt.% of water. The comparison of uptakes discloses that osmotic promoters possessing greater osmolality will permit a greater flux than those of lesser osmolality. In addition, the water absorption in the test utilizing a 1% sodium chloride solution is less inasmuch as the presence of the sodium chloride in the solution suppresses the difference in salt gradient between the interior of the packet and the absorbed solution, thus resulting in a lower rate of water absorption.

EXAMPLE IV

Hollow fibers of relatively large circumference were prepared from polymeric cellulose acetate. Lengths of the fiber were taken, one end sealed, and various amounts of lithium chloride placed inside the hollow fibers as the high osmolailty promoter. The remaining ends were sealed, and the osmotic absorbents formed were designated O, P, and Q. These long and thin packets were immersed in water for up to 90 minutes, periodically removed, patted dry, and weighed. The results of these tests are set forth in Table 5 below.

TABLE 5

| Sample Reference | Polymer WGT CA(g) | Promoter ID and WGT(g) | Fiber Circumference | Fiber Length | Time (min) | Uptake on Polymer Basis |
|---|---|---|---|---|---|---|
| O | 0.043 | LiCl; 0.074 | 20 mm | 7.0 cm | 0 | 0% |
| | | | | | 30 | 2128% |
| | | | | | 60 | 3170% |
| | | | | | 90 | 3979% |
| P | 0.056 | LiCl; 0.110 | 16 mm | 11.5 cm | 0 | 0% |
| | | | | | 30 | 2404% |
| | | | | | 60 | 3212% |
| Q | 0.070 | LiCl; 0.112 | 22 mm | 10.0 cm | 0 | 0% |
| | | | | | 30 | 2313% |
| | | | | | 60 | 3416% |
| | | | | | 90 | 4180% |

**Claims**

1. An osmotic absorbent comprising an encapsulated semipermeable polymeric membrane shell containing therein a high osmolality promoter.

2. The osmotic absorbent of Claim 1 in which said semipermeable polymeric membrane shell has reverse osmosis or hyperfiltration properties.

3. The osmotic absorbent of Claim 1 in which said osmality promoter has a molecular weight under 500.

4. The osmotic absorbent of Claim 1 in which the weight ratio of polymeric membrane to promoter is in a range of from about 2:1 to about 1:10.

5. The osmotic absorbent of Claim 3 in which said osmolality promoter is selected from salts of metals of Group IA or IIA of the Periodic Table.

6. The osmotic absorbent as set forth in Claim 5 in which said salt is sodium chloride.

7. The osmotic absorbent as set forth in Claim 5 in which said salt is lithium chloride.

8. The osmotic absorbent of Claim 3 in which said high osmolality promoter is glucose.

9. The osmotic absorbent of Claim 3 in which said high osmolality promoter is sucrose.

10. The osmotic absorbent of Claim 1 in which said semipermeable polymeric membrane is cellulose acetate.

11. The osmotic absorbent of Claim 1 in which the thickness of said semipermeable polymeric membrane is in a range of from about 5 to about 30 microns.

| | | |
|---|---|---|
| European Patent Office | EUROPEAN SEARCH REPORT | Application Number |
| | | EP 89 30 9528 |

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4 686 776 (M. MATSUBARA) * Abstract; figure; claims 1,3,10; column 2, lines 15-28; column 3, lines 11-19 * | 1,3,4,8 ,11 | B 01 D 61/00 B 01 J 20/00 // A 61 L 15/00 F 26 B 5/16 |
| X | WO-A-8 302 054 (C. KAMME) * Abstract; figures 1,2; claims 1,6,9; page 1, lines 3-8; page 2, line 29 - page 3, line 23; page 4, lines 22-29; page 6, lines 1-15 * | 1,4,10, 11 | |
| X | PATENT ABSTRACTS OF JAPAN, vol. 12, no. 159 (C-495)[3006], 14th May 1988, page 86 C 495; JP-A-62 272 934 (SHOWA DENKO K.K.) 27-11-1987 * Abstract * | 1,4,11 | |
| X | FR-A-2 606 027 (LIVE INT. CO. LTD) * Abstract; figures; claims 1,7,8,12; page 2, lines 9-21; page 3, line 33 - page 4, line 15 * | 1,3,5-9 ,11 | |
| X | EP-A-0 011 798 (K. STACHE) * Abstract; claim 1; figures 1,2; page 4, line 3 - page 5, line 1; page 6, line 1 - page 7, line 6; page 9, line 5 * | 1-4,10, 11 | TECHNICAL FIELDS SEARCHED (Int. Cl.5) B 01 D |
| X | US-A-3 880 164 (N.H. STEPNO) * Abstract; column 3, line 41 - column 4, line 2; column 4, line 58 - column 5, line 14; column 7, lines 33-50; claim 1 * | 1-7,10, 11 | |

The present search report has been drawn up for all claims

| Place of search | Date of completion of the search | Examiner |
|---|---|---|
| THE HAGUE | 05-12-1989 | HOORNAERT P.G.R.J. |